# EUROPEAN PATENT SPECIFICATION

(11) **EP 1 373 254 B1**
(45) Date of publication and mention of the grant of the patent: **02.11.2005**
(21) Application number: 01271106.5
(22) Date of filing: 06.12.2001
(51) Int. Cl.: C07D 403/06, A61K 31/40

(54) **Process for the preparation of the anti-migraine drug eletriptan**
Prozess zur Herstellung des Anti-Migräne Wirkstoffs Eletriptan
Procédé pour la préparation de l'anti-migraine médicament eletriptan

(30) Priority: 20.12.2000 GB 0031094
(43) Date of publication of application: 02.01.2004
(73) Proprietor: Pfizer Limited, Sandwich, Kent CT13 9NJ (GB); PFIZER INC., New York, N.Y. 10017 (US)
(72) Inventor: OGILVIE, Ronald J., Pfizer Global Res&Development, Sandwich, Kent CT13 9NJ (GB)
(74) Representative: Ruddock, Keith Stephen
(86) International application number: PCT/IB2001/002338
(87) International publication number: WO 2002/050063

(56) References cited:
- WO-A-92/06973
- KOJIMA S ET AL: "Fluorescent Properties of Model Chromophores of Tyrosine-66 Substituted Mutants of Aequorea Green Fluorescent Protein (GFP)" TETRAHEDRON LETTERS, ELSEVIER SCIENCE PUBLISHERS, AMSTERDAM, NL, vol. 39, no. 29, 16 July 1998 (1998-07-16), pages 5239-5242, XP004162428 ISSN: 0040-4039
- WILLEMS, E. ET AL. : "Porcine carotid vascular effects of eletriptan (UK-116,044): a new 5-HT1b/1D receptor agonist with anti-migraine activity" NAUNYN-SCHMIEDEBERG'S ARCH PHARMACOL, vol. 358, no. 2, 1998, page 212-219 XP002189843

## Description

The present invention is concerned with an improved process for the preparation of the anti-migraine drug, (R)-5-(2-benzenesulphonylethyl)-3-N-methylpyrrolidin-2-ylmethyl)-1H-indole (eletriptan), available commercially as the hydrobromide salt: 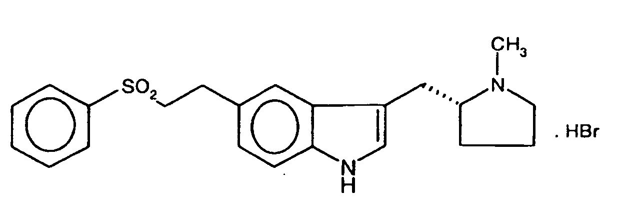 and with an intermediate in the process.

European Patent No. 0592438 describes the preparation of eletriptan by the catalytic reduction of (R)-5-(2-benzenesulphonylethenyl)-3-(N-methylpyrrolidin-2-ylmethyl)-1H-indole, which compound is prepared by (i) reacting N-benzyloxycarbonyl-D-proline acid chloride with 5-bromoindole in the presence of a Grignard reagent, (ii) reducing the resulting (R)-3-(N-benzyloxycarbonylpyrrolidin-2-ylcarbonyl)-5-bromo-1H-indole to give (R)-5-bromo-3-(N-methylpyrrolidin-2-ylmethyl)-1H-indole and (iii) reacting same with phenyl vinyl sulphone in the presence of a palladium catalyst, a triarylphosphine and a base.

The complete sequence may be represented as follows:

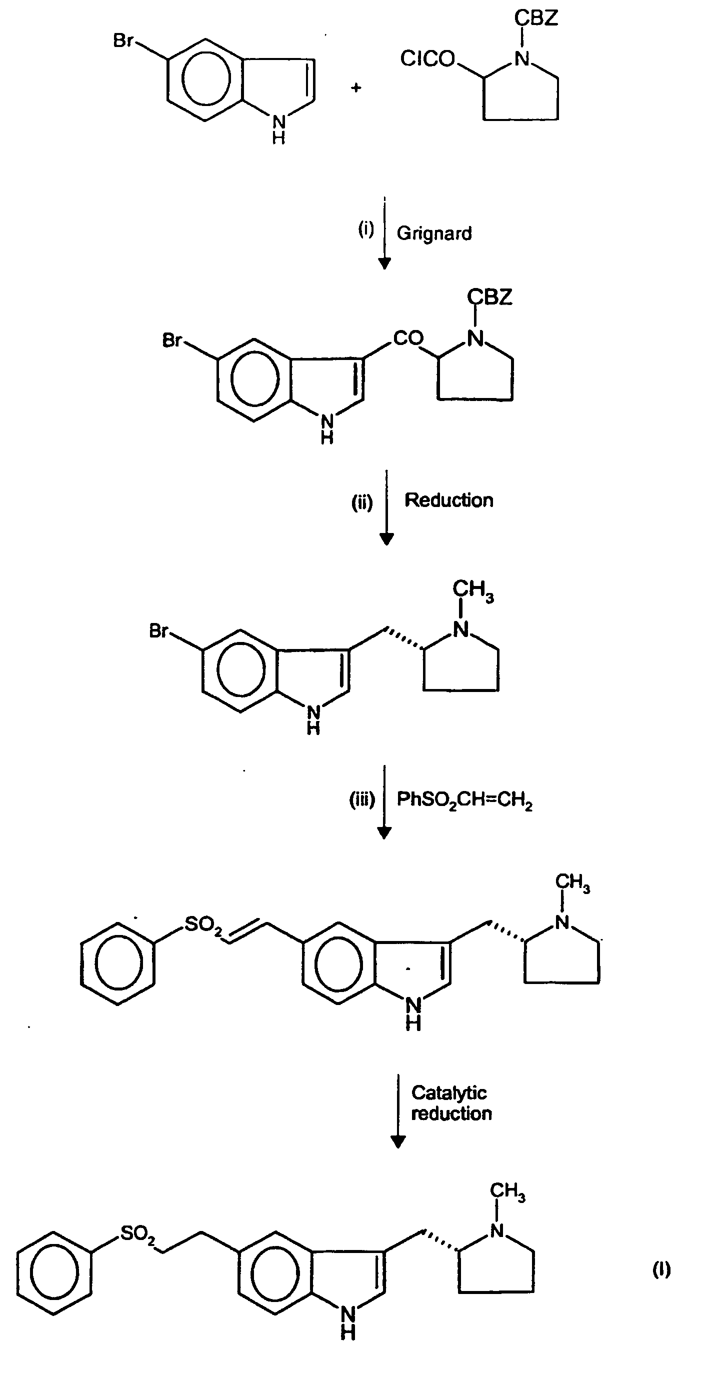

While the foregoing sequence produces eletriptan of formula (I) in reasonable yield, it has been found that the (R)-5-(2-benzenesulphonylethenyl)-3-(N-methylpyrrolidin-2-ylmethyl)-1H-indole precursor is prone to dimerise when attempting to recrystallise in impure form and/or drying prior to catalytic reduction:

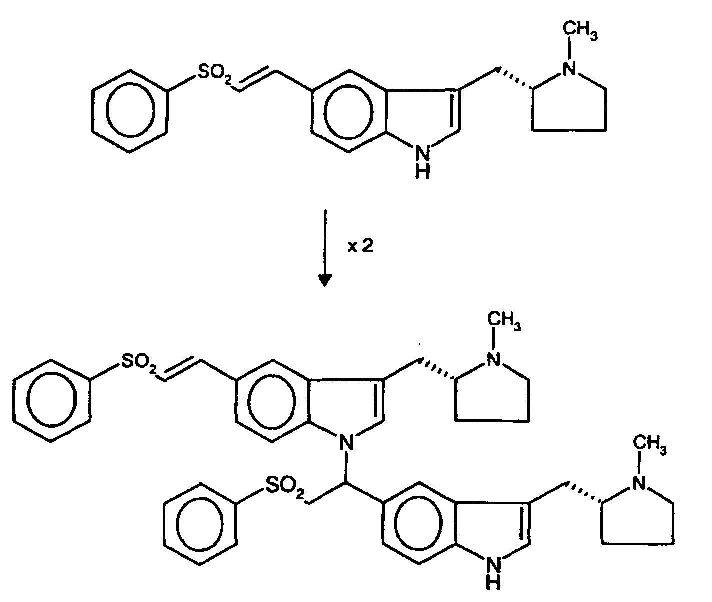

Not only does the formation of this dimeric impurity reduce the yield of eletriptan, but, perhaps more importantly, it requires the costly and time-consuming removal of said dimer in order to provide hydrobromide salt of sufficient purity to meet the stringent standards required for regulatory approval.

As a result of this difficulty, we have now developed an alternative route to eletriptan which avoids the use of a precursor which is prone to dimerisation. Specifically, the process of the invention comprises the preparation of eletriptan by the hydrolysis of (R)-1-acetyl-5-(2-benzenesulphonylethyl)-3-(N-methylpyrrolidin-2-ylmethyl)-1H-indole, which compound may conveniently be prepared by (i) N-acetylating (R)-5-bromo-3-(N-methylpyrrolidin-2-ylmethyl)-1H-indole, (ii) reacting the resulting (R)-1-acetyl-5-bromo-3-(N-methylpyrrolidin-2-ylmethyl)-1H-indole with phenyl vinyl sulphone in the presence of a palladium catalyst, a triarylphosphine and a base to give (R)-1-acetyl-5-(2-benzenesulphonylethenyl)-3-(N-methylpyrrolidin-2-ylmethyl)-1 H-indole and (iii) catalytically reducing same.

The complete sequence may be represented as follows:

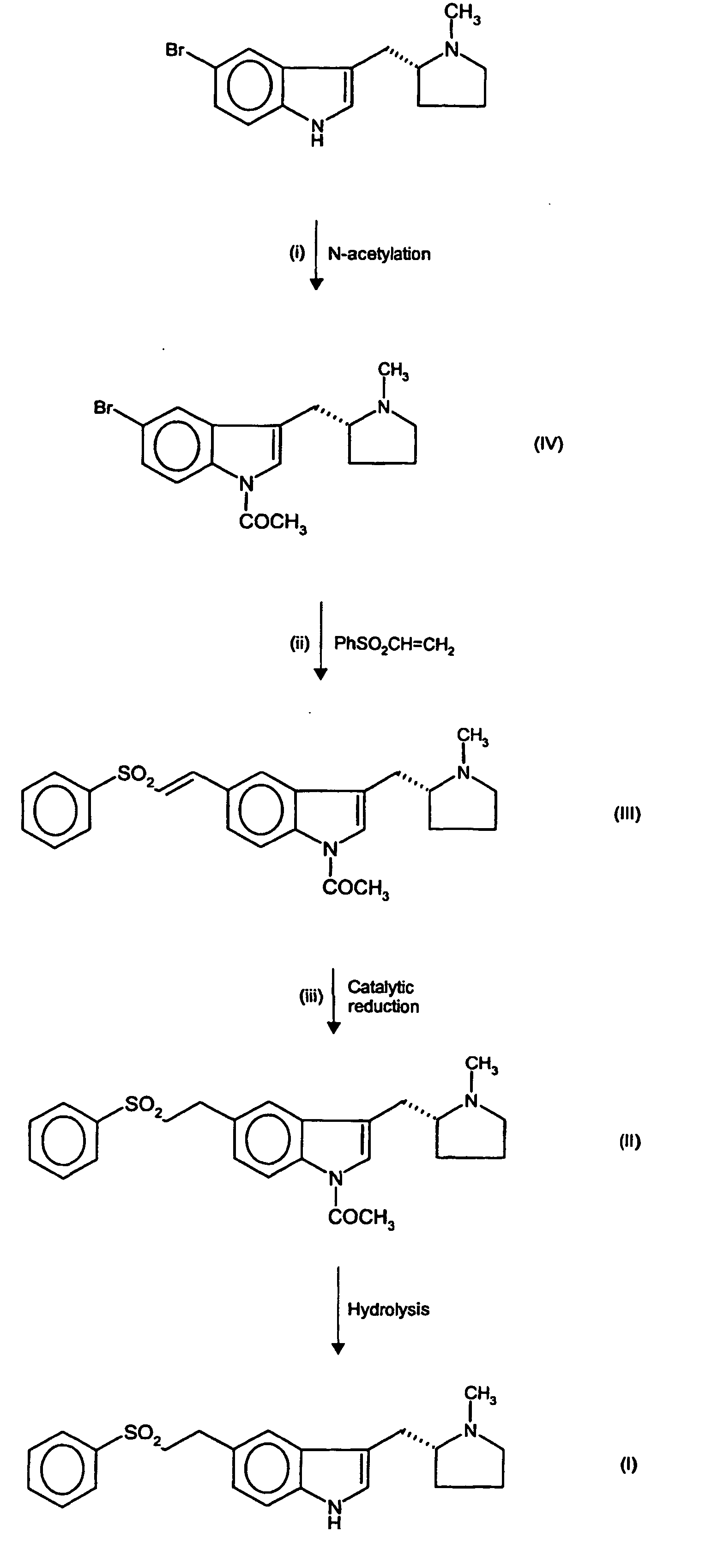

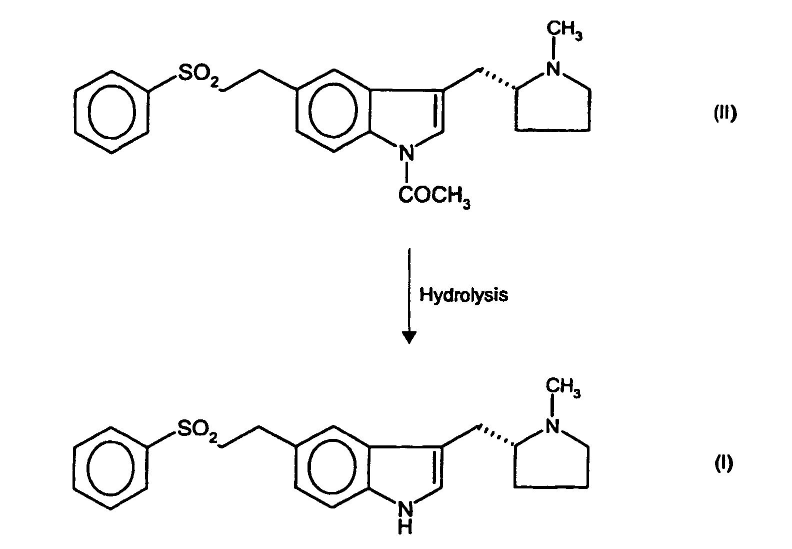

By the use of this process, it is possible to avoid the formation of unwanted dimer and thereby obtain eletriptan of high purity in good yield without the subsequent costly and time-consuming purification steps needed to remove the dimeric impurity.

Thus according to the present invention, there is provided a process for the preparation of a compound of formula (I) which comprises hydrolysis of a compound of formula (II), typically under basic conditions, more especially potassium carbonate in methanol/water.

According to another aspect of the invention, the compound of formula (II) used in the process may be obtained by catalytic reduction of a compound of formula (III), typically using hydrogen or a hydrogen source in the presence of a suitable catalyst. The reduction is typically carried out using hydrogen at a pressure of from 1 to 15 atmospheres or using a hydrogen source such as ammonium formate or formic acid. Suitable catalysts include palladium on carbon, for example, 5% w/w Pd/C, Raney nickel, platinum oxide, rhodium, or ruthenium. The reduction is conveniently carried out in the presence of an acid, for example, methanesulphonic acid, acetic acid, or trifluoroacetic acid. The compound of formula (II) so obtained is conveniently slurried with cold aqueous tetrahydrofuran before hydrolysis to the compound of formula (I).

The invention specifically provides the aforementioned compound of formula (II) which has not hitherto been described.

According to yet another aspect of the invention, the compound of formula (III) used in the process may be obtained by treating a compound of formula (IV) with phenyl vinyl sulphone in the presence of a palladium catalyst, a triarylphosphine and a base in accordance with the process described in Example 57 of US Patent 5,607,951.

According to yet a further aspect of the invention, the compound of formula (IV) used in the process may be obtained by the N-acetylation of (R)-5-bromo-3-(N-methylpyrrolidin-2-ylmethyl)-1H-indole, also in accordance with the process described in Example 57 of aforementioned US Patent No. 5,607,951.

Eletriptan obtained by the process of the invention may be converted to a pharmaceutically acceptable acid addition salt by treatment with an appropriate acid; said conversion may conveniently be carried out *in situ* without isolation of the compound of formula (I). A particularly preferred salt is the hydrobromide obtained by treatment with hydrobromic acid.

### EXAMPLE

The process of the invention may be illustrated by the following example of the preparation of (R)-5-(2-benzenesulphonylethyl)-3-N-methylpyrrolidin-2-ylmethyl)-1H-indole (I) and its hydrobromide salt:

### (a) Preparation of (R)-1-acetyl-5-(2-benzenesulphonylethyl)-3-(N-methylpyrrolidin-2-ylmethyl)-1H-indole (II)

To a solution of the compound of formula (III) (200g) prepared by the method described in Example 57 of aforementioned US Patent No. 5,607,951 in acetone (2.0L) was added water (0.5L). Methanesulphonic acid (43.2g, 0.95 equiv.) was added dropwise and the resulting solution stirred for 5 minutes before adding 5% w/w Pd/C catalyst (89.0g, Johnson Mattey Type 58, 50% wet). The solution was hydrogenated at room temperature at 200psi hydrogen for 18 hours.

The catalyst was removed by filtration and the filtrate stripped to give an acetone-free slurry. To this was added dropwise 40% aqu. NaOH (30mL) followed by water (1.5L). The resulting slurry was stirred for 20 minutes and further 40% aqu. NaOH (20mL) added. After granulation for 2 hours under vigorous stirring, the suspension was filtered and sucked dry for 30 minutes to give a beige damp solid which was either
(i) dried at 45°C to give the desired product (193.0g, yield 95%) or
(ii) taken up in tetrahydrofuran (1.6L) to which was added water (1.5L in total) over 10 minutes. The resulting suspension was stirred vigorously for 18 hours, filtered and sucked dry for 30 minutes to give the desired product as a beige damp solid (corrected weight 129.0g, yield 67%). Either form may be used directly in step (b):

### (b) Preparation of (R)-5-(2-benzenesulphonylethyl)-3-N-methylpyrrolidin-2-ylmethyl)-1H-indole (I)

To a solution of the compound of formula (II) (95.9g) from step (a)(i) or (ii) in acetone (1 L) and methanol (0.1L) was added K₂CO₃ (46.8g, 1.5 equiv.) and the resulting mixture stirred for 24 hours. To this was added charcoal (50g) followed an hour later by anhy. MgSO₄ (300g). The resulting suspension was stirred for 1 hour and filtered. The filtrate was stripped to give a damp solid which was dried *in vacuo* at 45°C to give the desired product (79.3g, 91.8%).

In the case where the compound of formula (I) is to be converted to a pharmaceutically acceptable acid addition salt, isolation of the compound of formula (I) may be avoided by directly treating the solution obtained by hydrolysis with the appropriate acid, for example, hydrobromic acid to give the hydrobromide salt:

### (c) Preparation of (R)-5-(2-benzenesulphonylethyl)-3-N-methylpyrrolidin-2-ylmethyl)-1H-indole (I) and in situ hydrobromination thereof

To a solution of the compound of formula (II) (95.9g) from step (a)(i) or (ii) in acetone (1L) and methanol (0.1L) was added K₂CO₃ (46.8g, 1.5 equiv.) and the resulting mixture stirred for 24 hours. To this was added charcoal (50g) followed an hour later by anhy. MgSO₄ (300g). The resulting suspension was stirred for 1 hour and filtered.

The filtrate was partially concentrated by azeotropic distillation to remove methanol and the volume readjusted to 0.45L with acetone. A solution of 48% w/v HBr (33.2g, 0.95 equiv.) in acetone (50mL) was added dropwise and the resulting suspension stirred for 72 hours. This was filtered, sucked dry for 30 minutes and dried *in vacuo* at 45°C to give the desired product as slightly beige crystals (71.8g, 68.5%).

In a preferred embodiment of the invention, certain steps may be 'telescoped' in order to reduce handling and accelerate processing time. For example, as indicated in step (a)(ii), drying the compound of formula (II) prior to hydrolysis may be avoided by using damp material slurried in aqueous tetrahydrofuran. Likewise, as indicated in step (c), isolation of the compound of formula (I) before conversion to a salt may be avoided by forming the salt *in situ*.

## Claims

1. A process for the preparation of a compound of formula (I) 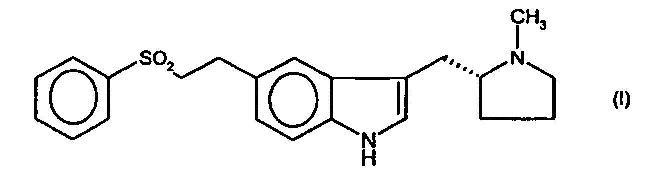 which comprises hydrolysis of a compound of formula (II)

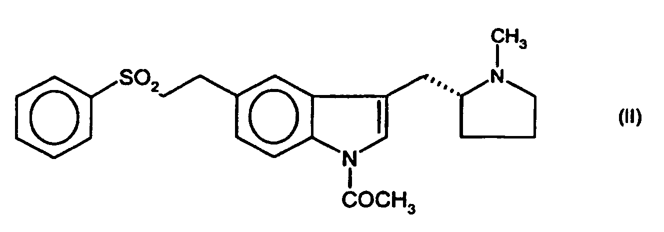

2. A process according to Claim 1 which is carried out under basic conditions.

3. A process according to Claim 2 wherein said hydrolysis is performed using potassium carbonate in methanol/water.

4. A process according to any of Claims 1 to 3 wherein the compound of formula (II) is obtained by catalytic reduction of a compound of formula (III)

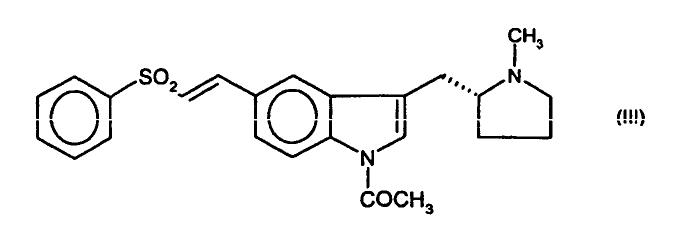

5. A process according to Claim 4 wherein said reduction is carried out using hydrogen or a hydrogen source in the presence of a suitable catalyst.

6. A process according to Claim 5 wherein said reduction is carried out using hydrogen at a pressure of from 1 to 15 atmospheres.

7. A process according to Claim 5 wherein said reduction is carried out using a hydrogen source which is ammonium formate or formic acid.

8. A process according to according to any of Claims 4 to 7 wherein said catalyst is palladium on carbon, Raney nickel, platinum oxide, rhodium, or ruthenium.

9. A process according to Claim 8 wherein said catalyst is 5% w/w palladium on carbon.

10. A process according to any of Claims 4 to 9 wherein the catalytic reduction is carried out in the presence of an acid.

11. A process according to Claim 10 wherein said acid is methanesulphonic acid, acetic acid, or trifluoroacetic acid.

12. A process according to any of Claims 4 to 11 wherein the compound of formula (II) obtained by catalytic reduction is slurried with cold aqueous tetrahydrofuran before hydrolysis to the compound of formula (I).

13. A process according to any of Claims 4 to 12 wherein the compound of formula (III) is obtained by treating a compound of formula (IV) 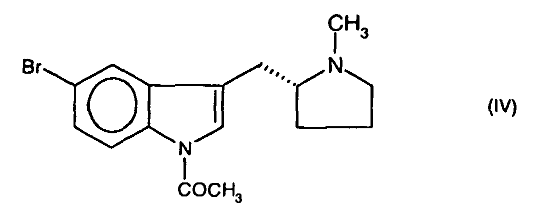 with phenyl vinyl sulphone in the presence of a palladium catalyst, a triarylphosphine and a base.

14. A process according to Claim 13 wherein the compound of formula (IV) is obtained by N-acetylating (R)-5-bromo-3-(N-methylpyrrolidin-2-ylmethyl)-1H-indole.

15. A process according to any of Claims 1 to 14 wherein the compound of formula (I) so obtained is converted to a pharmaceutically acceptable acid addition salt by treatment with an appropriate acid.

16. A process according to Claim 15 wherein said conversion is carried out *in situ* without isolation of the compound of formula (I).

17. A process according to Claim 15 or 16 wherein the acid is hydrobromic acid and the resulting salt is the hydrobromide.

18. The compound of formula (II):

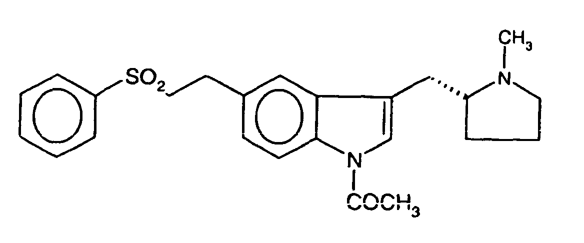

## Patentansprüche

1. Verfahren zur Herstellung einer Verbindung der Formel (I) 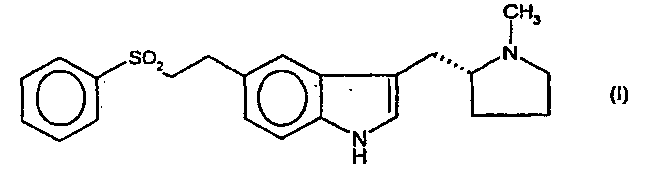 das die Hydrolyse einer Verbindung der Formel (II) 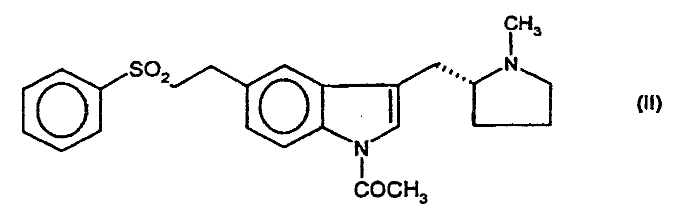 umfasst.

2. Verfahren nach Anspruch 1, das unter basischen Bedingungen durchgeführt wird.

3. Verfahren nach Anspruch 2, wobei die Hydrolyse unter Verwendung von Kaliumcarbonat in Methanol/Wasser durchgeführt wird.

4. Verfahren nach einem der Ansprüche 1 bis 3, wobei die Verbindung der Formel (II) durch katalytische Reduktion einer Verbindung der Formel (III) 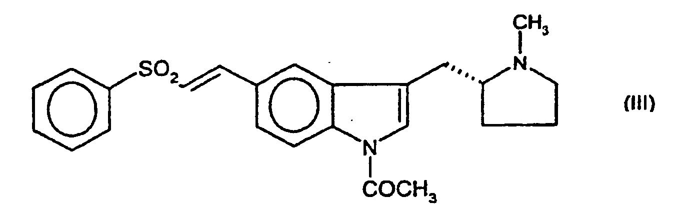 erhalten wird.

5. Verfahren nach Anspruch 4, wobei die Reduktion unter Verwendung von Wasserstoff oder einer Wasserstoffquelle in Gegenwart eines geeigneten Katalysators durchgeführt wird.

6. Verfahren nach Anspruch 5, wobei die Reduktion unter Verwendung von Wasserstoff bei einem Druck von 1 bis 15 Atmosphären durchgeführt wird.

7. Verfahren nach Anspruch 5, wobei die Reduktion unter Verwendung einer Wasserstoffquelle, die Ammoniumformiat oder Ameisensäure ist, durchgeführt wird.

8. Verfahren nach einem der Ansprüche 4 bis 7, wobei der Katalysator Palladium-auf-Kohle, Raney-Nickel, Platinoxid, Rhodium oder Ruthenium ist.

9. Verfahren nach Anspruch 8, wobei der Katalysator 5 % (Gew/Gew) Palladium-auf-Kohle ist.

10. Verfahren nach einem der Ansprüche 4 bis 9, wobei die katalytische Reduktion in Gegenwart einer Säure durchgeführt wird.

11. Verfahren nach Anspruch 10, wobei die Säure Methansulfonsäure, Essigsäure oder Trifluoressigsäure ist.

12. Verfahren nach einem der Ansprüche 4 bis 11, wobei die durch katalytische Reduktion erhaltene Verbindung der Formel (II) vor der Hydrolyse zur Verbindung der Formel (I) mit kaltem wässrigem Tetrahydrofuran aufgeschlämmt wird.

13. Verfahren nach einem der Ansprüche 4 bis 12, wobei die Verbindung der Formel (III) durch Behandeln einer Verbindung der Formel (IV) 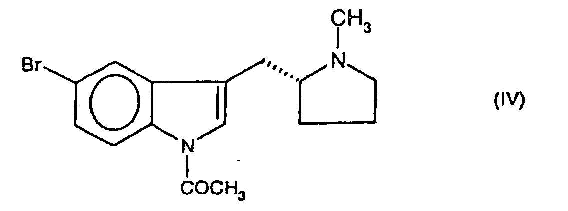 mit Phenylvinylsulfon in Gegenwart eines Palladiumkatalysators, eines Triarylphosphins und einer Base erhalten wird.

14. Verfahren nach Anspruch 13, wobei die Verbindung der Formel (IV) durch N-Acetylierung von (R)-5-Brom-3-(N-methylpyrrolidin-2-ylmethyl)-1H-indol erhalten wird.

15. Verfahren nach einem der Ansprüche 1 bis 14, wobei die so erhaltene Verbindung der Formel (I) durch eine Behandlung mit einer geeigneten Säure in ein pharmazeutisch akzeptables Säureadditionssalz umgewandelt wird.

16. Verfahren nach Anspruch 15, wobei die Umwandlung in situ ohne Isolieren der Verbindung der Formel (I) durchgeführt wird.

17. Verfahren nach Anspruch 15 oder 16, wobei die Säure Bromwasserstoffsäure ist und das gebildete Salz das Hydrobromid ist.

18. Die Verbindung der Formel (II):

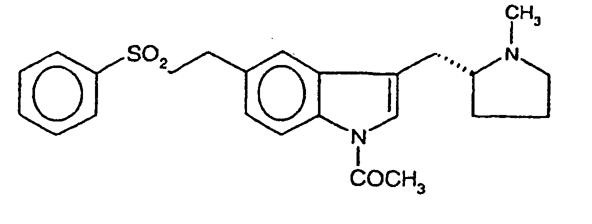

## Revendications

1. Procédé de préparation d'un composé de formule (I) 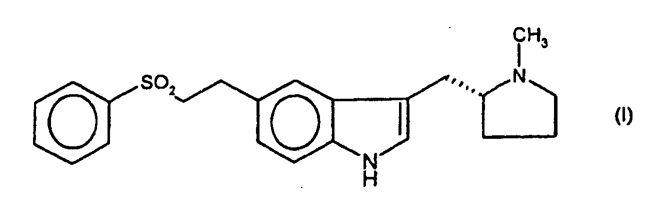 qui comprend l'hydrolyse d'un composé de formule (II)

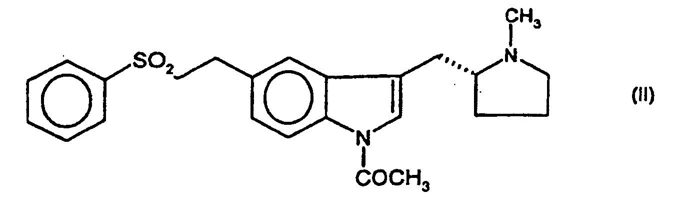

2. Procédé selon la revendication 1, qui est mis en oeuvre dans des conditions basiques.

3. Procédé selon la revendication 2, dans lequel ladite hydrolyse est mise en oeuvre en utilisant du carbonate de potassium dans du méthanol/eau.

4. Procédé selon l'une quelconque des revendications 1 à 3, dans lequel le composé de formule (II) est obtenu par réduction catalytique d'un composé de formule (III)

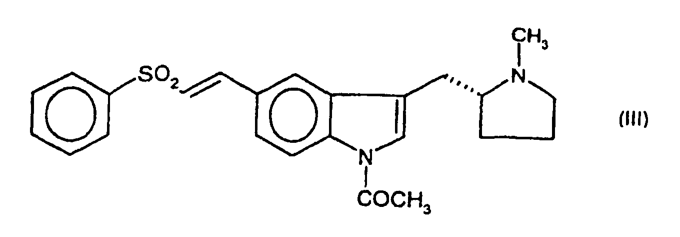

5. Procédé selon la revendication 4, dans lequel ladite réduction est mise en oeuvre en utilisant de l'hydrogène ou une source d'hydrogène en présence d'un catalyseur convenable.

6. Procédé selon la revendication 5, dans lequel ladite réduction est mise en oeuvre en utilisant de l'hydrogène à une pression comprise entre 1 et 15 atmosphères.

7. Procédé selon la revendication 5, dans lequel ladite réduction est mise en oeuvre en utilisant une source d'hydrogène qui est du formiate d'ammonium ou de l'acide formique.

8. Procédé selon l'une quelconque des revendications 4 à 7, dans lequel ledit catalyseur est du palladium sur carbone, du nickel de Raney, de l'oxyde de platine, du rhodium ou du ruthénium.

9. Procédé selon la revendication 8, dans lequel ledit catalyseur est du palladium à 5% (poids/poids) sur carbone.

10. Procédé selon l'une quelconque des revendications 4 à 9, dans lequel ladite réduction catalytique est mise en oeuvre en présence d'un acide.

11. Procédé selon la revendication 10, dans lequel ledit acide est l'acide méthanesulfonique, l'acide acétique ou l'acide trifluoroacétique.

12. Procédé selon l'une quelconque des revendications 4 à 11, dans lequel le composé de formule (II) obtenu par réduction catalytique est mis en bouillie avec du tétrahydrofurane froid avant l'hydrolyse en composé de formule (I).

13. Procédé selon l'une quelconque des revendications 4 à 12, dans lequel le composé de formule (III) est obtenu par traitement d'un composé de formule (IV) 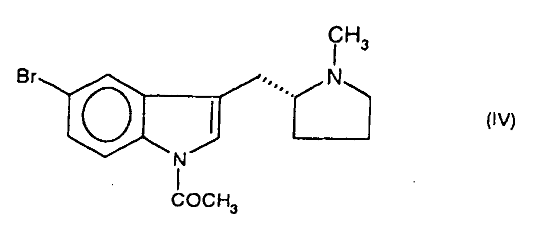 avec de la phénylvinylsulfone en présence d'un catalyseur au palladium, une triarylphosphine et une base.

14. Procédé selon la revendication 13, dans lequel le composé de formule (IV) est obtenu par N-acétylation du (R)-5-bromo-3-(N-méthylpyrrolidin-2-ylméthyl)-1H-indole.

15. Procédé selon l'une quelconque des revendications 1 à 14, dans lequel le composé de formule (I) ainsi obtenu est transformé en un sel d'addition acide pharmaceutiquement acceptable par traitement avec un acide approprié.

16. Procédé selon la revendication 15, dans lequel ladite transformation est mise en oeuvre *in situ* sans isolement du composé de formule (II).

17. Procédé selon la revendication 15 ou 16, dans lequel l'acide est l'acide bromhydrique et le sel résultant est le bromhydrate.

18. Composé de formule (II) :
